Europäisches Patentamt

(19) European Patent Office

Office européen des brevets

(11) Publication number: **0 105 413**
**A2**

## (12) EUROPEAN PATENT APPLICATION

(21) Application number: **83109420.6**

(22) Date of filing: **22.09.83**

(51) Int. Cl.³: **C 12 Q 1/68**

(30) Priority: **30.09.82 US 428526**

(43) Date of publication of application:
**18.04.84 Bulletin 84/16**

(84) Designated Contracting States:
**CH DE FR GB IT LI SE**

(71) Applicant: **MILES LABORATORIES INC.**
**1127 Myrtle Street**
**Elkhart Indiana 46514(US)**

(72) Inventor: **Morris, David Lindsay**
**23274 Ronda Drive**
**Elkhart, IN 46514(US)**

(74) Representative: **Jesse, Ralf-Rüdiger, Dr. et al,**
**Bayer AG Zentralbereich Patente Marken und Lizenzen**
**D-5090 Leverkusen 1 Bayerwerk(DE)**

(54) Cell detection by measurement of flavin adenine dinucleotide.

(57) A method for detecting cellular material, e.g., microbial cells or debris from ruptured microbial cells, in a liquid test sample by determining flavin adenine dinucleotide (FAD) in the liquid sample. Comparing the amount of FAD determined in the test sample to that present in a reference sample, e.g., containing no significant amount of relevant cellular material, enables detection of cellular material of interest in the sample. The method is particularly applicable to the detection of microbial contamination in biological fluids and is preferably carried out by measuring FAD with an apo(glucose oxidase) assay composition. Test systems, kits and devices are also provided.

EP 0 105 413 A2

## CELL DETECTION BY MEASUREMENT
## OF FLAVIN ADENINE DINUCLEOTIDE

### BACKGROUND OF THE INVENTION

#### 1. *FIELD OF THE INVENTION*

The invention relates to methods and means for detecting cellular material, e.g., somatic or microbial cells or cellular debris, in liquids such as biological fluids. In particular, the invention relates to a method and means for detecting cellular material by measuring the amount of the cofactor flavin adenine dinucleotide (FAD) present in a test sample of the liquid.

#### 2. *DESCRIPTION OF THE PRIOR ART*

Classical techniques for detecting cells involve time consuming, labor intensive culture methods in which any cells present in a test sample are grown to the point that cell colonies can be visualized, enumerated, and otherwise manipulated for additional testing. Despite many drawbacks, the classical culture methods remain in general use in the detection and identification of cells, particularly bacterial cells, because of the lack of a simpler alternative.

MS-1250

- 2 -

A much studied indirect method for detecting cells, again bacterial cells in particular, involves the detection of the enzyme cofactor adenosine triphosphate (ATP). In this method, cellular ATP is released by cell rupture and measured by an enzymatic bioluminescent assay. The basis of the assay is the fire fly bioluminescence reaction involving the action of the enzyme luciferase on its substrate luciferin in the presence of ATP and other critical cofactors. Representative of this method are the teachings of U.S. Pat. Nos. 3,745,090; 3,971,703; and 4,283,490 and *Methods in Enzymology*, vol. 57(1978) pp. 65-72. A major disadvantage of the ATP bioluminescence technique is the requirement for a luminescence detection instrument which is not generally found in clinical laboratories.

It is generally known that there exist a variety of intracellular enzyme cofactors other than ATP, including nicotinamide adenine dinucleotide (NAD) and its phosphorlyated derivative (NADP), pyridoxyl phosphate (PLP), tetrahydrofolic acid, thiamine pyrophosphate (TPP), coenzyme A (CoA), biotin, heme-containing molecules, metal ions such as magnesium, zinc, and manganese, and flavin adenine dinucleotide (FAD) [*Principles of Bacteriology, Virology, and Immunity,* Wilson and Miles, Edward Arnold (Publishers) Ltd. (London) 6th ed. 1975]. Only limited use has been made of these other cofactors, principally NAD, in detecting cells and cellular material in test liquids.

The interaction of FAD with apo(glucose oxidase) was studied by Swoboda, *Biochim. Biophys. Acta 175*: 365-387 (1969).

MS-1250

- 3 -

The use of FAD as a label in specific binding
assays, e.g., homogeneous competitive binding immuno-
assays, is described in U.S. Pat. No. 4,238,565,
assigned to the present assignee.

## SUMMARY OF THE INVENTION

It has now been found that cellular material can
be simply and advantageously detected in liquid samples
by measuring flavin adenine dinucleotide (FAD). Accordingly, the invention is applicable to a wide variety
of analyses, and is especially useful in the detection
of microbial contamination in biological fluids by
comparing the amount of FAD in a test sample with the
FAD content of an uncontaminated sample.

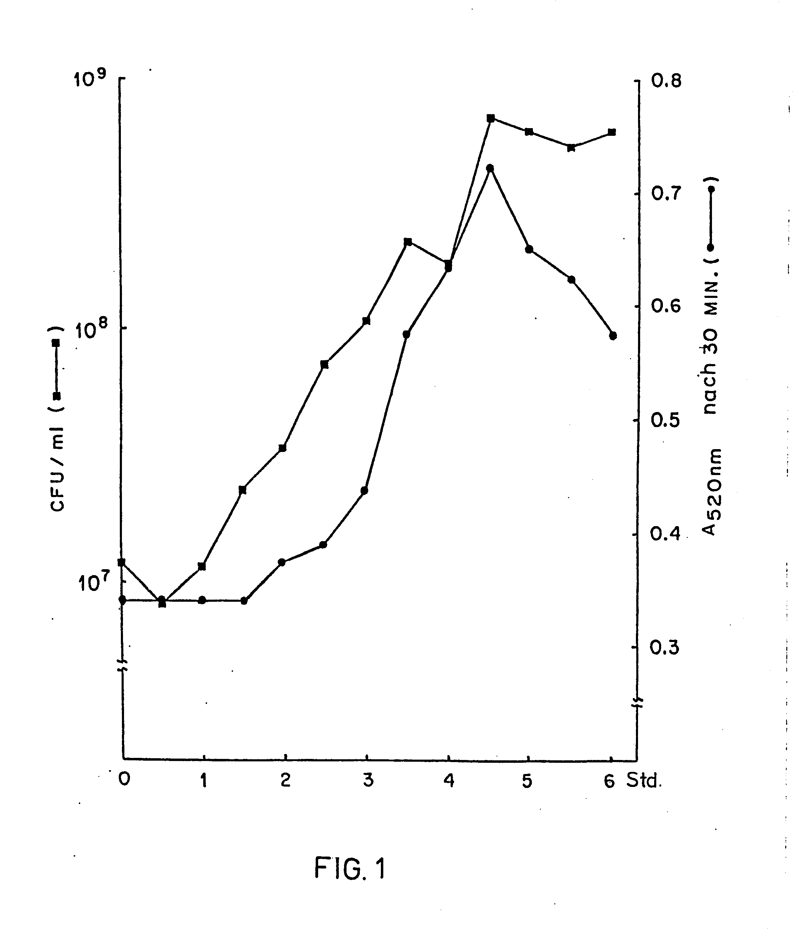

## BRIEF DESCRIPTION OF THE DRAWING

The drawing is a graphical representation of the
correlation between an FAD assay method and a conventional colony enumeration method in the analysis of
time sequenced test samples taken during the growth of
an *E. coli* culture.

## DESCRIPTION OF THE PREFERRED EMBODIMENTS

The present invention involves two principal
steps: (1) determination of FAD in the liquid test
sample and (2) comparison of such measured amount with
a reference standard representing the amount of FAD in
a reference sample. If the liquid under assay is a
biological fluid, the reference sample may or may not
contain some measurable FAD background value. Where
the biological fluid normally contains some FAD, some
cut-off value for normal FAD content will be established

MS-

- 4 -

against which the FAD content of the test sample will be compared. Additional FAD in the test sample will indicate the presence of abnormal cellular material in the sample. Where the biological fluid normally contains no measurable FAD (i.e., the reference sample contains no significant amounts of cellular material), the reference FAD value will be essentially zero and the determination of measurable FAD in the test sample will of itself indicate in such case the presence of cellular contamination in the test sample.

Somatic or microbial cells are detectable by the present method, as well as the debris from ruptured cells. It has been found that viable cells can be detected with or without special treatment of the test sample to rupture the cells. If desired, rupture of cells can be accomplished by conventional means such as sonication or chemical or enzymatic lysis, e.g., treatment with detergent. In general, rupture of the cells in the test sample will release additional intracellular FAD so that a more sensitive assay is possible.

A principal application of the present invention is to the detection of microbial cells or microbial contamination in liquids, particularly biological fluids of clinical and diagnostic significance. By microbial contamination is intended whole viable or nonviable microbial cells (including all microorganisms, from prokaryotes, principally bacteria, to eukaryotes such as protozoa, fungi and yeast) and debris from the rupture of such cells. It may be important to detect the presence of whole microbial cells in the test sample, or whether there were whole cells in the test sample at some point prior to the actual assay (which

- 5 -

would be evidenced by the presence of cell debris and released intracellular FAD), or both.  Thus, contamination is a term used herein to describe cellular material not normally found in the liquid under assay, and includes cell debris as well as whole cells.

Liquids and fluids that can be assayed according to the present invention include industrial fluids, waste and commercial effluents and the like, environmentally important liquids, such as surface and well water, and fluids of scientific or medical importance, principally biological fluids such as the body fluids of man and animals.

In accordance with the present invention, FAD can be determined or measured in the liquid test sample by any available means including fluorometric methods [Burch *et al, J. Biol. Chem. 175*:457 (1948); Koziol, *Methods in Enzymology 3(Part B)*:253(1971); and Mayhew and Wassink, *Methods in Enzymology 66(Part E)*:217 (1980)], polarographic methods [Knoblock, *Methods in Enzymology 3(Part B)*:305(1971)]; spectrophotometric methods [Fazehus and Kokai, *Methods in Enzymology 3 (Part B)*:385(1971)]; and in general any method available to one working in the field.

In the usual case, the present method is accomplished by contacting the test sample with a chemical test system which provides a detectable signal related to FAD concentration and comparing the measured signal to a reference signal representing the signal provided by the chemical test system upon contact with the reference sample.  As indicated above, such reference sample contains an amount of FAD which is considered to be

MS-1250

- 6 -

normal for the purposes of the assay. Where the liquid under assay normally contains no significant amount of cellular material of interest, the reference sample will reflect such situation and the reference signal will essentially be the background signal, if any, of the chemical test system.

The detectable signal that is generated can be any physical, chemical or electrical phenomenon which is sensible by human observation or instrument detection. Electrochemical, calorimetric, manometric and like signals may be generated. Preferably, optical signals will be selected and include fluorescence, phosphorescence, chemiluminescence, color changes due to changes of light absorption in the visible, ultraviolet or infra red regions of the spectrum.

It is especially preferred to employ a chemical test system comprising (a) an apoenzyme which combines with FAD to form a holoenzyme (a catalytically active enzyme), (b) substrate for the holoenzyme, and (c) an indicator which provides the detectable signal in the presence of a product of the enzymatic reaction of the substrate. Useful apoenzymes include apo(xanthine oxidase) [Sugiura *et al*, *Chem. Pharm. Bull* 29:1361(1981)], apo(amino acid oxidase) [Warburg and Christian, *Biochem. Z.* 298:150(1938); and see *Biochem. J.* 33:2008(1939), *J. Biol. Chem.* 136:177(1940), and *Methods in Enzymology* 3:950(1957)], apo(glutathione reductase) [Scott *et al*, *J. Biol. Chem.* 238:3928(1963) and Staal *et al*, *Biochim. Biophys. Acta* 185:39(1969)], and apo(lipoamide dehydrogenase) [Visser and Beeger, *Biochim. Biophys. Acta* 206:224(1970)]. Most preferred is apo(glucose oxidase). [Swoboda, *Biochim. Biophys. Acta* 175:365(1969) and U.S. Pat. No. 4,268,631]. Where the generated signal is optical in character, particularly where it is colorimetric (e.g., a color change), the present invention

MS-1250

provides an extremely simple and useful means for detecting cellular material. The most preferred apo(glucose oxidase) test system additionally comprises glucose as enzyme substrate, and, as the indicator, a peroxidatively active substance and a photogen which provides the optical signal.

Peroxidatively active substances are well known in the art and include substances from various organic and inorganic sources. Plant peroxidases, such as horseradish peroxidase or potato peroxidase, can be used. Inorganic compounds having peroxidase activity include iodides, such as sodium and ammonium iodides, and molybdates. In addition, urohemin and a number of other prophyrin substances having peroxidative activity can be used. Other substances which are not enzymes, but which have peroxidative activity include such compounds as iron sulfocyanate, iron tannate, ferrous ferrocyanide, potassium chromic sulfate and the like.

Likewise, a wide variety of useful photogens are known in the art to provide a detectable optical signal in the presence of or upon reaction with hydrogen peroxide and the peroxidatively active substance. Such photogens include chromogens or color indicator dyes, for example, *ortho*-tolidine, benzidine, syringaldazine, diaminofluorine, and tetramethylbenzidine, and related derivatives, and also include coupled dye systems such as those conventionally referred to a Trinder reagents, e.g., a phenol and 4-aminoantipyrine. Other useful photogens are fluorogenic peroxidase substrates such as scopoletin (6-methoxyumbelliferone), *para*-hydroxyphenylacetic acid, and various fluoroscein derivatives such as diacetyldichlorofluorescein (U.S. Pat. No. 4,269,938). Chemiluminescers such as luminol, isoluminol, pyragallol, and their various derivatives and analogs, can also be used as the photogen.

MS-1250

- 8 -

The test system can include other components as well such as buffers, diluents, stabilizers and the like. Also, where the rupture of cells or digestion of cellular debris is desired for performing the assay, detergents, proteolytic enzymes, and like materials may also be included in the test system.

The FAD-sensitive chemical test system can be in any convenient form such as a test kit, a test composition admixture, or a test device. The test composition admixture or the test kit components can be liquid, usually aqueous solutions; dry powders, tablets, or the like; or in the form of gels, films and so forth. A preferred test system format is the reagent strip test device wherein the test system is incorporated with a carrier member or matrix. The carrier may take the form of either bibulous or nonbibulous, porous or nonporous, matrices which are insoluble in and maintain their structural integrity when exposed to water or other fluids of analytical interest. Suitable bibulous matrices which can be used include absorbent paper, polymeric films and gels, cellulose, wood, synthetic resin fleeces, woven and nonwoven fabrics, and the like. Nonbibulous matrices include glass fiber and organoplastic materials, such as polypropylene and the like. The carrier matrix can be soaked, immersed in, sprayed or printed with the liquid reagent composition and the carrier matrix thereafter dried by suitable means, such as ambient or forced air drying to leave the dry reagent/matrix combination. The matrix can be advantageously affixed to an insoluble support member such as an organoplastic strip, e.g., polystyrene, by suitable means, such as double faced adhesive tape, for ease of use.

When in the form of an apo(glucose oxidase) reagent strip device, the present test system provides a most simple and convenient means for detecting cellular material. In contrast with the prior art culture methods and ATP-bioluminescence techniques, the present reagent strip device provides an assay result upon being immersed and withdrawn from the test sample. The generated optical signal is readily read by an instrument or where appropriate, such as where color changes are generated, observed by the technician. The reference signal can be stored within the instrument or can be in the form of an observable standard such as a color chip or chart, and the comparison made internally by the instrument or by the human observor. Such a simple, straight forward, rapid method for detecting cellular material, particularly microbial contamination, in liquids, particularly biological fluids, has not been available previously.

The present invention will now be illustrated, but is not intended to be limited, by the following examples.

### EXAMPLE 1

A characteristic growth curve may be obtained when a viable microorganism, e.g., bacterial, culture is inoculated into a suitable medium and the rate of growth is observed. For a visual representation of the growth curve, the concentration of organisms per milliliter is plotted against the time elapsed in hours.

Immediately after inoculation, some time is required for the cells to adjust to the new environment. During this period of adjustment, called the lag phase, the cells undergo considerable internal change but little or no cell division occurs. The next phase is the log or exponential phase. Cell division occurs at a constant rate determined by the nature of the organism and the environmental conditions. As the organisms exhaust the available nutrients and waste products accumulate, the stationary phase is reached. During this time, the viable organism count remains relatively constant with cell division and cell death occurring at the same rate. Finally, the stationary phase gives way to the death phase where cell death occurs at a rate greater than cell division.

A culture broth of *Escherichia coli* was grown over a period of six hours during which period aliquots were removed and samples inoculated on plates for enumeration of colonies. The remainder of each aliquot was frozen and later thawed and FAD determined.

Part I - Colony Enumeration

A.   Materials

1.   Prepared Tryptic Soy Agar (TSA) plates (Acu Media, Baltimore, MD, USA).

2.   Tryptic Soy Broth (TSB) (Acu Media) - 100 milliliter (ml)/bottle (preincubate at $35° \pm 2°C$ for one hour or more).

3.   *E. coli*, ATCC 8739 - 18 hr. suspension in TSB.

4.   Sterile Saline (0.85% NaCl) 9.9 ml/tube; 19.0 ml/tube.

5.   Micro pipettors - 0.01 ml; 0.1 ml (calibrated).

6.   Test tubes - 10 millimeter (mm) x 100 mm.

7.   Sterile pipettes - 1 ml, 2 ml, 10 ml.

- 11 -

8. Cryo vials (11).

9. Bent glass rod for plate spreading.

10. Container of Ice - for transporting filled cryo vial to freezer.

B. Procedure

1. An 18 hour *E. coli* suspension was adjusted to $1.0 \times 10^8$ CFU/ml (colony forming units per milliliter).

2. A volume of 10 ml of sterile TSB was withdrawn from the preincubated 100 ml flask.

   (a) A 1 ml volume was labelled "Preincubation Control" and frozen.

   (b) Three 0.1 ml aliquots were spread on TSA plates and incubated at $35° \pm 2°C$ for 48 hours.

3. A 10 ml volume of the standardized culture was pipetted into the remaining 90 ml TSB and mixed well resulting in a 1:10 dilution of the standardized culture.

4. A total of 2.51 ml was removed from the inoculated TSB.

   (a) A 1 ml volume was pipetted into a cryo vial, labeled with the time sampled and frozen.

   (b) A 1.5 ml volume was pipetted into a 10 mm x 100 mm test tube. The percent transmittance (%T) at 640 nanometers (nm) was read from a spectrophotometer and recorded.

   (c) A 0.01 ml volume was taken from the inoculated TSB and diluted with sterile saline to $10^{-3}$, $10^{-5}$ and $10^{-7}$. Triplicate 0.1 ml volumes of these dilutions were inoculated on spread plates for enumeration of colony forming units (CFU).

MS-1250

- 12 -

The following dilution scheme was used:

1. 0.01 ml culture + 10.1 ml saline ($10^{-3}$) spread plate 0.1 ml in triplicate ($10^{-4}$).

2. 0.1 ml ($10^{-3}$) + 9.9 ml saline ($10^{-5}$) spread plate 0.1 ml in triplicate ($10^{-6}$).

3. 0.1 ml ($10^{-5}$) + 9.9 ml saline ($10^{-7}$) spread plate 0.1 ml in triplicate ($10^{-8}$).

(d) All plates were incubated for 48 hours at $35° \pm 2°C$.

5. Step 4 was repeated at 30 minutes intervals for a total of six hours.

The results are summarized in Table 1.

Table 1

| Time (hours) | %T | Avg. CFU/ml |
|---|---|---|
| 0.0 | 98.5 | $1.4 \times 10^7$ |
| 0.5 | 99.0 | $9.0 \times 10^6$ |
| 1.0 | 97.0 | $1.2 \times 10^7$ |
| 1.5 | 95.0 | $3.0 \times 10^7$ |
| 2.0 | 94.5 | $4.4 \times 10^7$ |
| 2.5 | 86.0 | $1.1 \times 10^8$ |
| 3.0 | 82.0 | $1.6 \times 10^8$ |
| 3.5 | 71.0 | $3.6 \times 10^8$ |
| 4.0 | 66.0 | $2.9 \times 10^8$ |
| 4.5 | 60.0 | $1.2 \times 10^9$ |
| 5.0 | 54.0 | $1.0 \times 10^9$ |
| 5.5 | 49.5 | $8.7 \times 10^8$ |
| 6.0 | 49.5 | $1.0 \times 10^9$ |

Part II - FAD Determination

A.   Reagents

(a)   Apoglucose oxidase solution - 2 micromolar (μM) apoglucose oxidase (FAD-binding sites - see U.S. Pat. No. 4,268,631), 4 millimolar (mM) 4-aminoantipyrine, 30% (w/v) glycerol, 0.1% (w/v) bovine serum albumin, 0.02% (w/v) sodium azide, and 0.1 M sodium phosphate buffer, pH 7.0.

(b)   Apoglucose oxidase/anti(glucose oxidase) antiserum solution - 5 ml of above apoglucose oxidase solution mixed with 0.4 ml of anti(glucose oxidase) antiserum.

(c)   Glucose oxidase assay solution - 2.1 mM sodium 3,5-dichloro-2-hydroxybenzene sulfonate, 0.105 M glucose, 60 μg/ml horse-radish peroxidase (Sigma type III), and 0.1 M sodium phosphate buffer, pH 7.0.

B.   Preparation of sample

The frozen aliquots of growth medium were thawed and 0.1 ml diluted with 0.9 ml of ice cold 0.1 M sodium phosphate buffer, pH 7.0, containing 0.1% (w/v) sodium azide.  The diluted solutions were kept on ice.

- 14 -

C.    Procedure for assay

A 0.10 ml sample of the diluted growth medium was placed in each of a series of disposable plastic cuvettes and mixed with 1.90 ml of the glucose oxidase assay solution. The cuvettes were then incubated in a water bath thermostated at 30°C. Apoglucose oxidase/anti(glucose oxidase) antiserum solution (100 µl) was placed in cuvette caps and the assays were started by inserting the caps on top of the cuvettes and inverting several times to mix. The assay solutions were then incubated at 30°C for 30 minutes each and the absorbance at 520 nm recorded versus a blank consisting of the glucose oxidase assay solution above. An increase of 0.3 absorbance units would represent the production of about 1 nanomolar (nM) of FAD in the growth medium.

The results of the colony enumeration method and the FAD assay are shown in the drawing. A definite correlation was shown between the number of bacterial colonies and FAD concentration.

EXAMPLE 2

A second study was undertaken to investigate the effect of various sample treatments on the correlation of the FAD assay to bacterial contamination.

A.    Reagents

(a)    Reagent A - 4 µM apoglucose oxidase (measured in terms of FAD binding sites), 8 mM 4-aminoantipyrine, 50% (w/v) glycerol, and 0.1 M phosphate buffer, pH 7.0.

MS-1250

(b)  Reagent B - 8 µl of anti(glucose oxidase) antiserum added to each ml of Reagent A.

(c)  Reagent C - 2.1 mM 3,5-dichloro-2-hydroxybenzene sulfonate, 1.05 M glucose, 60 µg/ml peroxidase, and 0.1 M phosphate buffer, pH 7.0.

B.  Assay procedure

Assays were performed by mixing 0.05 ml of a sample with 1.90 ml of Reagent C in cuvettes and equilibrating to 25°C.  Reaction was started by adding 0.058 µl of Reagent B.  After 30 minutes of incubation at 25°C, the absorbance at 520 nm was read.

C.  Calibration curve

FAD samples were prepared in 0.1 M phosphate buffer, pH 7.0, and 50 µl were included in the assay. Absorbance at 520 nm correlated with FAD concentration as shown in Table 2.

Table 2

| FAD Concentration (pmoles/ml) | Absorbance at 520 nm at 30 minutes |
|---|---|
| 0 | 0.071 |
| 2.05 | 0.279 |
| 4.10 | 0.477 |
| 6.15 | 0.668 |
| 8.20 | 0.856 |
| 10.25 | 1.056 |
| 12.30 | 1.237 |

MS-1250

D.   Measurement of FAD in treated and untreated samples.

1.   Untreated samples

Thawed aliquots of the *E. coli* samples from Example 1 were diluted 10-fold with 1.0 M phosphate buffer, pH 7.0, and assayed.

2.   Sonicated samples

*E. coli* samples were sonicated using a Branson Sonicator (Model W140D, Heat Systems-Ultrasonics, Inc., Plainview, NY, USA), diluted 10-fold with buffer and assayed.

3.   Pepsin treated samples

Sonicated *E. coli* samples were digested by mixing 0.1 ml of 10-fold diluted sample with 0.2 ml of 0.2 M KCl/HCl, pH 1.1, containing 10 mg/ml pepsin.  After a 2 hour incubation at 37°C, the samples were assayed for FAD.

The results are shown in Table 3.

Table 3

| Time of Growth (hours) | CFU/ml | FAD content (pmoles/ml) | | |
|---|---|---|---|---|
| | | untreated | sonicated | sonicated and digested |
| 0 | $1.4 \times 10^7$ | 9 | 11 | 34 |
| 0.5 | $9.0 \times 10^6$ | 9 | 12 | 36 |
| 1.0 | $1.2 \times 10^7$ | 9 | 12 | 36 |
| 1.5 | $3.0 \times 10^7$ | 9 | 13 | 38 |
| 2.0 | $4.4 \times 10^7$ | 10 | 15 | 39 |
| 2.5 | $1.1 \times 10^8$ | 12 | 16 | 42 |
| 3.0 | $1.6 \times 10^8$ | 17 | 20 | 52 |
| 3.5 | $3.6 \times 10^8$ | 30 | 33 | 70 |
| 4.0 | $2.9 \times 10^8$ | 35 | 37 | 77 |
| 4.5 | $1.2 \times 10^9$ | 56 | 58 | 110 |
| 5.0 | $1.0 \times 10^9$ | 56 | 57 | 108 |
| 5.5 | $8.7 \times 10^8$ | 53 | 57 | 108 |
| 6.0 | $1.0 \times 10^9$ | 53 | 56 | 114 |

The data demonstrate that FAD content correlates with the level of bacterial growth in untreated and treated samples. Treatment of the sample to rupture cells results in higher FAD levels in the resulting assay.

MS-

WHAT IS CLAIMED IS:

1. A process for detecting cellular material in a liquid test sample, comprising the steps of determining the amount of flavin adenine dinucleotide in said sample and comparing said amount with the amount of flavin adenine dinucleotide present in a reference sample.

2. A process of Claim 1 wherein the test sample is contacted with a chemical test system which provides a detectable signal related to flavin adenine dinucleotide concentration and wherein such signal is compared to a reference signal representing the signal provided by said chemical test system upon contact with said reference sample.

3. A process of Claim 2 wherein said reference sample contains no significant amounts of cellular material.

4. A process of Claim 2 wherein said chemical test composition comprises (a) an apoenzyme which combines with flavin adenine dinucleotide to form a holoenzyme, (b) substrate for said holoenzyme, and (c) an indicator which provides said detectable signal in the presence of a product of the enzymatic reaction of said substrate.

5. The process of Claim 4 wherein said apoenzyme is apo(glucose oxidase), said substrate is glucose, and said indicator comprises a peroxidatively active substance and a photogen which provides an optical signal in the presence of hydrogen peroxide and said peroxidatively active substance.

6. The process of Claim 1 wherein said sample is first treated to rupture cells present therein.

7. The process of Claim 1 wherein said liquid test sample is a biological fluid.

8. A chemical test system for determining cellular material in a liquid test sample, comprising

(a) an apoenzyme which combines with flavin adenine dinucleotide to form a holoenzyme,
(b) substrate for said holoenzyme, and
(c) an indicator which provides a detectable signal in the presence of a product of the enzymatic reaction of said substrate,

in amounts substantially to optimize the signal produced in the presence of cellular material in said sample.

9. The test system of Claim 8 which additionally comprises a chemical lysing agent capable of rupturing cells present in said test sample.

10. A test device for determining cellular contamination in a liquid test sample, comprising the chemical test system of any one of Claims 8 or 9 and a carrier member incorporated therewith.

MS-1250

FIG. 1